# FASCICULE DE BREVET EUROPEEN

(11) **EP 3 222 225 B1**
(45) Date de publication et mention de la délivrance du brevet: **08.11.2023**
(21) Numéro de dépôt: 16162459.8
(22) Date de dépôt: 25.03.2016
(51) Int. Cl.: A61B 17/16, A61F 2/46

(54) **MANCHE PREHENSEUR DE RAPE FEMORALE**
GRIFF EINER FEMURRASPEL
HANDLE FOR GRIPPING A FEMORAL BONE RASP

(43) Date de publication de la demande: 27.09.2017
(73) Titulaire: Dedienne Sante, 34130 Mauguio (FR); AXIOM, 69004 Lyon (FR)
(72) Inventeur: BONIN, Nicolas, 69004 LYON (FR); MANIN, Christian, 69153 DECINES Cedex (FR); BLION, Florent, 69153 DECINES Cedex (FR); GAUME, Jean-Michel, 69153 Decines Cedex (FR)
(74) Mandataire: LLR

(56) Documents cités:
- WO-A1-94/08528
- FR-A1- 2 770 128
- US-A- 5 792 143

## Description

La présente invention concerne un manche préhenseur de râpe fémorale.

De plus, la présente invention concerne aussi un instrument de préparation d'un fémur lors de la pose d'une prothèse de hanche.

Traditionnellement, lors d'une opération de pose d'une prothèse fémorale, le praticien réalise une planification radiographique de la prothèse fémorale en prenant comme référence le centre de la tête prothétique de la prothèse fémorale.

Durant toute l'opération, le praticien peut utiliser le sommet du grand trochanter comme référence anatomique, pour positionner le centre de la tête prothétique.

Généralement, pour implanter une prothèse fémorale, le praticien réalise une coupe à la base du fémur.

Cette coupe est souvent effectuée au-dessus du sommet du petit trochanter, selon un plan incliné par rapport à l'axe longitudinal du fémur.

La coupe ainsi réalisée, permet au praticien d'accéder au canal médullaire du fémur.

Le praticien peut ensuite conformer le canal intra-médullaire en utilisant successivement des râpes fémorales de tailles croissantes, jusqu'à la taille de la prothèse fémorale planifiée.

Les râpes fémorales présentent la géométrie d'une tige de prothèse fémorale, et sont accouplées successivement à un manche préhenseur permettant au praticien de manipuler chaque râpe successivement. Cependant, il est difficile pour le praticien d'apprécier si l'aménagement du logement dans le fémur est correct en termes de profondeur ; cette appréciation se fait au jugé et fait largement appel à l'expérience du praticien.

La publication de demande de brevet N° FR 2 770 128 décrit un dispositif de mesure incluant un bloc apte à être fixé sur un embout d'une râpe fémorale et un palpeur apte à être engagé au travers de ce bloc. Ce bloc et ce palpeur font partie d'un dispositif de vérification permettant de vérifier, après la préparation du fémur et avant la mise en place de l'implant fémoral, la position future qu'occupera l'implant. Lorsque le praticien pense avoir réalisé correctement le logement de la prothèse, quant à la forme finale prédéterminée de celui-ci et à la position du plan de résection, il procède à une première vérification géométrique visant ce logement, et par conséquent la position future de l'implant fémoral sélectionné, au moyen dudit dispositif de vérification.

Le matériel selon ce document ne permet donc pas à un praticien d'apprécier, en cours de réalisation du logement dans le fémur, si ce logement est correct en termes de profondeur. De plus, si la profondeur détectée n'est pas adéquate, ce matériel va impliquer au moins un démontage du manche, un montage du bloc, un démontage du bloc et un remontage du manche, qui a pour conséquence de nombreuses manipulations supplémentaires et un allongement notable de la durée de l'intervention.

Une autre solution connue, consiste à repérer le centre de tête prothétique sur le manche préhenseur. Cependant cette dernière solution nécessite d'intégrer l'ensemble des positions de centre de tête d'une même gamme de tiges prothétiques. Or, une même gamme de tiges prothétiques comprend différentes tailles et angulations du col fémoral, ce qui peut rendre le repérage illisible et être source de confusion pour le chirurgien.

Dans ce contexte technique, la présente invention a pour but de fournir un manche préhenseur de râpe fémorale qui permette d'évaluer, clairement et en continu, la position du centre de tête prothétique et l'enfouissement de la râpe fémorale dans le canal médullaire.

Selon une définition générale, l'invention concerne un manche préhenseur destiné à être assemblé à une râpe fémorale. Le manche préhenseur comprend, aménagés sur lui, des moyens de matérialisation de position de la râpe fémorale par rapport au fémur. Les moyens de matérialisation comprennent au moins une portion d'appui apte à venir en regard du sommet du grand trochanter et au moins une portion de matérialisation apte à être immobilisée par rapport au manche préhenseur dans la direction longitudinale de ce manche, de façon à matérialiser au moins un point appartenant au plan tangent au sommet du grand trochanter lorsque la portion d'appui prend appui sur le sommet du grand trochanter.

L'inventeur a en effet pu constater que les râpes fémorales existantes n'intègrent pas de portion qui matérialise, en cours de l'aménagement du logement dans le fémur, en référence au sommet du grand trochanter et donc en référence au centre de tête prothétique, le bon enfouissement de la râpe fémorale dans le canal médullaire, ce qui ne permet pas à un praticien d'évaluer ce bon enfouissement en cours dudit aménagement du logement dans le fémur.

L'inventeur a alors pu concevoir d'aménager lesdits moyens de matérialisation sur le manche préhenseur, de telle sorte que ces moyens de matérialisation puissent être utilisés quelle que soit la taille de râpe utilisée.

Ainsi, le manche préhenseur de râpe selon l'invention permet de visualiser aisément au moins un point appartenant au plan tangent au sommet du grand trochanter. De manière générale, le centre de tête prothétique est positionné dans le plan tangent au sommet du grand trochanter. La matérialisation d'au moins un point appartenant au plan tangent au sommet du grand trochanter permet donc au praticien de visualiser clairement la position du centre de tête prothétique. De plus, la portion d'appui permet au praticien d'évaluer clairement l'enfouissement de la râpe fémorale.

Selon un mode de réalisation, les moyens de matérialisation comprennent au moins un organe de repérage. Ledit au moins un organe de repérage peut présenter une portion d'appui et une portion de matérialisation.

De plus, ledit au moins un organe de repérage peut être est configuré pour être monté de manière amovible sur le manche préhenseur.

Ainsi, l'organe de repérage peut être séparé du manche préhenseur pour faciliter le rangement du manche préhenseur.

Le manche préhenseur peut comprendre au moins un logement débouchant, transversal à la direction longitudinale du manche préhenseur, ayant une section transversale telle qu'il est apte à recevoir ledit au moins un organe de repérage de manière ajustée mais amovible.

De manière préférentielle, le manche préhenseur comprend au moins trois logements débouchants, décalés selon une direction de décalage parallèle à la direction longitudinale du manche préhenseur.

Le manche préhenseur est ainsi configuré de telle sorte qu'en condition d'utilisation, la direction de décalage soit parallèle à l'axe du fémur.

Il s'agit là d'une disposition particulièrement avantageuse de l'invention. En effet, le décalage des logements débouchants permet au manche préhenseur, accouplé aux différentes râpes, de matérialiser différentes positions de centres de têtes prothétiques.

Ainsi, le manche préhenseur permet à un praticien d'adapter la position de l'organe de repérage en fonction de la variété de la prothèse dont il souhaite matérialiser la position du centre de tête prothétique.

Le manche préhenseur peut présenter un bossage qui comprend ledit au moins un logement débouchant.

Selon un mode de réalisation, ledit au moins un organe de repérage est monobloc et configuré pour que, lorsque ledit au moins un organe de repérage est monté de manière amovible au manche préhenseur fixé à une râpe fémorale positionnée dans le canal médullaire d'un fémur, l'axe longitudinal dudit au moins un organe de repérage soit sensiblement perpendiculaire à l'axe du fémur.

En outre, ledit au moins un organe de repérage peut présenter une section circulaire.

De plus, ledit au moins un organe de repérage peut présenter sensiblement une forme en L qui comprend une portion préhensible.

Ainsi, un praticien peut aisément manipuler l'organe de repérage.

La présente invention concerne aussi un instrument de préparation d'un fémur qui comprend un manche préhenseur selon l'invention et une râpe fémorale.

D'autres caractéristiques et avantages de l'invention se dégageront de la description qui va suivre, en regard des dessins annexés qui représentent une forme de réalisation de l'invention.
- la figure 1 est une vue de face présentant côte-à-côte un instrument de préparation d'un fémur qui comprend un manche préhenseur selon l'invention, et un premier type de prothèse de hanche implantée dans un fémur.
- la figure 2 est une vue de face présentant côte-à-côte un instrument de préparation d'un fémur qui comprend un manche préhenseur selon l'invention, et un second type de prothèse de hanche implantée dans un fémur.

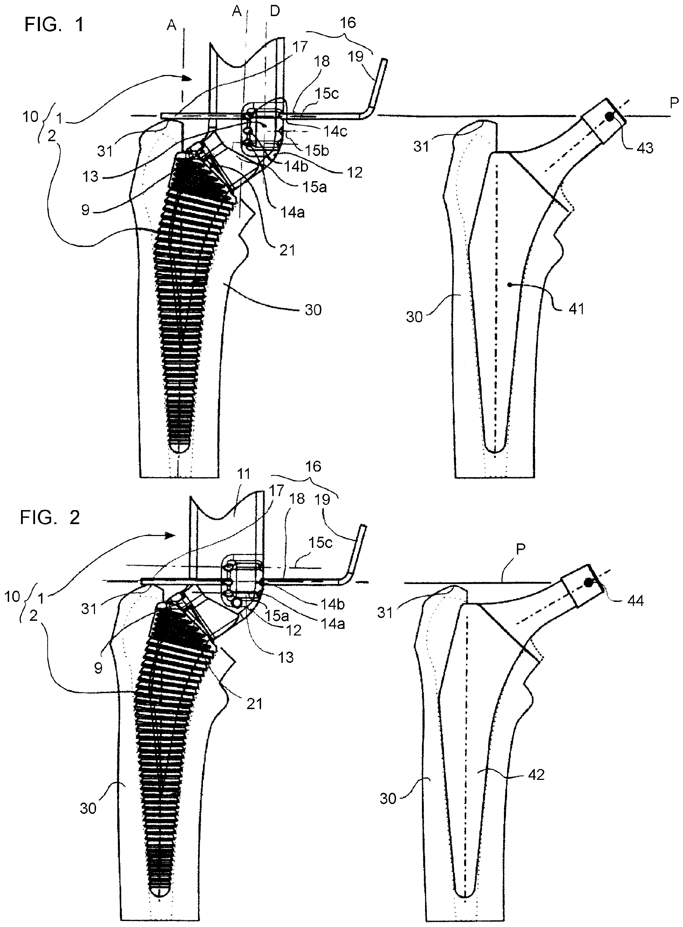

L'instrument de préparation 10 d'un fémur 30 représenté sur les figures 1 et 2 est introduit dans un fémur 30 qui comprend un canal médullaire et un grand trochanter présentant un sommet 31.

L'instrument de préparation 10 comprend un manche préhenseur 1 et une râpe fémorale 2.

La râpe fémorale 2 est connue en elle-même et présente la forme d'une tige fémorale prothétique munie d'une surface abrasive.

De plus, la râpe fémorale 2 présente des éléments de fixation qui sont positionnés selon un plan incliné par rapport à un axe longitudinal A de la râpe fémorale 2.

La région délimitant les éléments de fixation est pourvue d'une hauteur compensatoire 21 spécifique pour chacune des râpes fémorales 2 Cette hauteur compensatoire 21 est justifiée par les variétés de la prothèse fémorale. Il est précisé que lorsque la râpe fémorale 2 est introduite dans le canal médullaire du fémur 30, l'axe longitudinal A est sensiblement confondu avec l'axe du fémur.

Le manche préhenseur 1 comprend un corps 11, des moyens de matérialisation d'un point appartenant au plan P tangent au sommet 31 du grand trochanter, un coude 12, et des moyens de fixation à la râpe fémorale 2.

Le corps 11 est conçu pour permettre au praticien de tenir le manche préhenseur 1 et d'imprimer un mouvement à la râpe fémorale 2 lorsque le manche préhenseur 1 est fixé à la râpe fémorale 2.

Les moyens de fixation à la râpe fémorale 2 peuvent comprendre, par exemple, une nervure 9 et un élément encliquetable non visible sur les figures 1 et 2.

Le coude 12 est positionné entre le corps 11 et les moyens de fixation à la râpe fémorale 2 pour permettre une différence d'orientation entre le corps 11 et les moyens de fixation à la râpe fémorale 2.

De cette manière, lorsque le manche préhenseur 1 est fixé à la râpe fémorale 2, le coude 12 permet au corps 11 d'être orienté selon un axe A'.

Les moyens de matérialisation d'un point appartenant au plan tangent P au sommet 31 du grand trochanter comprennent un bossage 13 positionné sur le corps 11.

Selon le mode de réalisation ici présenté, le bossage 13 comprend trois logements débouchants 14a-14b-14c.

Les trois logements débouchants 14a-14b-14c présentent chacun un axe respectif 15a-15b-15c. Les axes 15a-15b-15c des logements débouchants 14a-14b-14c sont sensiblement perpendiculaires à un axe longitudinal de la râpe fémorale 2.

A l'usage, les axes 15a-15b-15c des logements débouchants 14a-14b-14c sont sensiblement perpendiculaires à l'axe A du fémur 30.

De plus, les logements débouchants 14a-14b-14c, sont décalés le long d'un axe de décalage D sensiblement parallèle à l'axe longitudinal du corps 11, et donc sensiblement parallèle à l'axe A du fémur 30.

De manière avantageuse, le corps 11 présente deux bossages 13 comprenant chacun des logements débouchants 14a-14b-14c. Les deux bossages 13 peuvent être configurés pour s'étendre de part et d'autre du corps 11, en condition d'utilisation. Ainsi, le manche préhenseur 1 peut être indifféremment utilisé pour préparer le fémur droit ou le fémur gauche d'un patient.

En outre, les logements débouchants 14a-14b-14c ont des sections transversales ajustées à un diamètre que présente un organe de repérage 16, afin d'être aptes chacun à accueillir ce dernier de manière ajustée mais amovible. L'organe de repérage 16 présente la forme d'une tige en forme de L monobloc.

L'organe de repérage 16 comprend une portion d'appui 17, une portion de matérialisation 18 et une portion préhensible 19.

Selon un mode de réalisation, le logement débouchant 14a est positionné le long de l'axe D, pour permettre de matérialiser un point appartenant au plan tangent P au sommet du grand trochanter auquel appartiendrait le centre d'une tête prothétique d'une prothèse varisée d'une taille comprise entre 1 et 5, donc une prothèse de taille comparativement inférieure dans une gamme de prothèses de différentes tailles.

Selon un même mode de réalisation, le logement débouchant 14b est positionné le long de l'axe D, pour permettre de matérialiser un point appartenant au plan tangent P au sommet du grand trochanter auquel appartiendrait le centre d'une tête prothétique d'une prothèse varisée d'une taille comprise entre 6 et 10, donc une prothèse de taille comparativement supérieure dans une gamme de tailles de prothèses, ou d'une prothèse standard d'une taille comprise entre 1 et 5.

Selon un même mode de réalisation, le logement débouchant 14c est positionné le long de l'axe D, pour permettre de matérialiser un point appartenant au plan tangent P au sommet du grand trochanter auquel appartiendrait le centre d'une tête prothétique d'une prothèse standard d'une taille comprise entre 6 et 10.

La combinaison des logements débouchants 14a-14b-14c du manche préhenseur 1 avec la hauteur compensatoire 21 de la râpe finale permet de simuler avec précision la position du centre de tête prothétique de l'implant planifié.

La figure 1 présente, côte à côte, l'instrument de préparation 10 introduit dans un fémur 30, et une prothèse de hanche standard 41 d'une taille comprise entre 6 et 10 introduite dans un fémur 30.

La prothèse de hanche standard 41 présente un centre de tête 5 prothétique 43.

La figure 2 présente côte-à-côte, l'instrument de préparation 10 introduit dans un fémur 30, et une prothèse de hanche varisée 42 d'une taille comprise entre 6 et 10 introduite dans un fémur 30.

La prothèse de hanche varisée 42 présente un centre de tête prothétique 44.

A l'usage, le praticien détermine, en fonction de l'anatomie du patient opéré, la taille et la variété de la prothèse de hanche qui va être implantée.

S'il juge approprié, par exemple, d'implanter une prothèse standard ayant une taille comprise entre 6 et 10, le praticien introduit l'organe de repérage 16 dans le logement débouchant 14c (voir figure 1).

De même, s'il juge approprié, par exemple, d'implanter une prothèse varisée ayant une taille comprise entre 6 et 10, le praticien introduit l'organe de repérage 16 dans le logement débouchant 14b (voir figure 2).

La portion préhensible 19 de l'organe de repérage 16 permet au praticien de manipuler aisément l'organe de repérage 16 pour l'introduire dans le logement débouchant 14a-14b-14c de son choix.

Lorsque l'organe de repérage 16 est introduit dans un logement débouchant 14a, 14b ou 14c, l'organe de repérage 16 définit un axe sensiblement perpendiculaire au corps 11 et donc, lors de l'utilisation, à l'axe A du fémur. Une fois que l'organe de repérage 16 est introduit dans le logement débouchant 14a, 14b, ou 14c, correspondant à la prothèse de hanche qui va être posée, le praticien peut utiliser l'instrument de préparation 10 pour conformer le canal médullaire du fémur 30.

Le praticien utilise l'instrument de préparation 10 en saisissant le corps 11 pour imprimer un mouvement permettant à la râpe fémorale 2 de râper le canal médullaire.

Lorsque le praticien utilise l'instrument de préparation 10, la portion d'appui 17 de l'organe de repérage 16 est positionnée en regard du sommet 31 du grand trochanter. Au fur et à mesure de la conformation du canal médullaire par la râpe fémorale 2, la portion d'appui 17 se rapproche du sommet 31 du grand trochanter.

Lorsque la portion d'appui 17 entre en contact avec le sommet 31 du grand trochanter, la portion de matérialisation 18 matérialise un point appartenant au plan tangent P au sommet du grand trochanter.

De plus, en appuyant sur le sommet 31 du grand trochanter, la portion d'appui 17 peut remplir une fonction de limitation d'enfouissement de la râpe fémorale 2 dans le canal médullaire du fémur 30. Le praticien peut alors arrêter de conformer le canal médullaire du fémur 30.

La portion de matérialisation 18 permet au praticien de visualiser le plan auquel appartient le centre de tête prothétique 43-44 appartenant à la prothèse de hanche 41-42 de son choix.

Comme on peut l'observer sur les figures 1 et 2, de manière particulièrement avantageuse, le décalage entre les logements débouchants 14a-14b-14c combiné à la hauteur compensatoire 21 de la râpe fémorale, permet à l'invention de correspondre à un grand nombre de prothèse de hanche.

Ainsi, l'invention propose un manche préhenseur comprenant des moyens de matérialisation qui permettent à un praticien d'évaluer en continu la position du centre de tête prothétique et l'enfouissement de la râpe fémorale.

Bien entendu l'invention ne se limite pas à la seule forme de réalisation représentée ci-dessus, elle embrasse au contraire toutes les formes de réalisations.

## Revendications

1. Manche préhenseur (1) destiné à être assemblé à une râpe fémorale (2), le manche préhenseur (1) étant apte à être saisi par un praticien pour imprimer un mouvement permettant à la râpe fémorale de râper un canal médullaire d'un fémur et comprenant, aménagés sur lui, des moyens de matérialisation de position de la râpe fémorale (2) par rapport au fémur, ces moyens de matérialisation comprenant au moins une portion d'appui (17) apte à venir en regard du sommet (31) du grand trochanter et au moins une portion de matérialisation (18) apte à être immobilisée par rapport au manche préhenseur dans la direction longitudinale de ce manche, de façon à matérialiser au moins un point appartenant au plan tangent (P) au sommet du grand trochanter lorsque la portion d'appui (17) prend appui sur le sommet (31) du grand trochanter.

2. Manche préhenseur (1) selon la revendication 1, **caractérisé en ce que** les moyens de matérialisation comprennent au moins un organe de repérage (16), ledit au moins un organe de repérage présentant la portion d'appui (17) et la portion de matérialisation (18).

3. Manche préhenseur (1) selon la revendication 2, **caractérisé en ce que** ledit au moins un organe de repérage (16) est configuré pour être monté de manière amovible sur le manche préhenseur (1).

4. Manche préhenseur (1) selon l'une des revendications 2 et 3, **caractérisé en ce que** le manche préhenseur (1) comprend au moins un logement débouchant (14a-14b-14c) transversal à la direction longitudinale du manche préhenseur, ayant une section transversale telle qu'il est apte à recevoir ledit au moins un organe de repérage (16) de manière ajustée mais amovible.

5. Manche préhenseur (1) selon la revendication 4, **caractérisé en ce que** le manche préhenseur (1) comprend au moins trois logements débouchants (14a-14b-14c), décalés selon une direction de décalage (D) parallèle à la direction longitudinale du manche préhenseur.

6. Manche préhenseur (1) selon la revendication 4 ou la revendication 5, **caractérisé en ce que** le manche préhenseur (1) présente un bossage (13) qui comprend ledit au moins un logement débouchant (14a-14b-14c).

7. Manche préhenseur (1) selon l'une des revendications 2 à 6, **caractérisé en ce que** ledit au moins un organe de repérage (16) est monobloc et configuré pour que, lorsque ledit au moins un organe de repérage (16) est monté de manière amovible au manche préhenseur (1) fixé à une râpe fémorale (2) positionnée dans le canal médullaire d'un fémur (30), l'axe longitudinal dudit au moins un organe de repérage (16) soit sensiblement perpendiculaire à l'axe du fémur (30).

8. Manche préhenseur (1) selon la revendication 7, **caractérisé en ce que** ledit au moins un organe de repérage (16) présente une section circulaire.

9. Manche préhenseur (1) selon la revendication 6, **caractérisé en ce que** ledit au moins un organe de repérage (16) présente sensiblement une forme en Lqui comprend une portion préhensible (19).

10. Instrument de préparation (10) d'un fémur (30) **caractérisé en ce qu'**il comprend un manche préhenseur (1) selon l'une des revendications 1 à 9 et une râpe fémorale (2).

## Patentansprüche

1. Griff (1), der dazu bestimmt ist, mit einer Femurraspel (2) zusammengebaut zu werden, wobei der Griff (1) von einem Arzt ergriffen werden kann, um eine Bewegung zu erzeugen, die es der Femurraspel ermöglicht, einen Markkanal eines Oberschenkelknochens zu raspeln, und auf ihm angeordnete Mittel zur Markierung der Position der Femurraspel (2) in Bezug auf den Oberschenkelknochen aufweist, wobei diese Markierungsmittel mindestens einen Auflageabschnitt (17), der geeignet ist, gegenüber dem Scheitel (31) des Trochanters major zu gelangen, und mindestens einen Markierungsabschnitt (18), der ausgestaltet ist, in Bezug auf den Griff in der Längsrichtung dieses Griffs unbeweglich zu sein, aufweisen, um mindestens einen Punkt zu markieren, der zur Tangentialebene (P) des Scheitels des Trochanters major gehört, wenn sich der Auflageabschnitt (17) auf dem Scheitel (31) des Trochanters major abstützt.

2. Griff (1) nach Anspruch 1, **dadurch gekennzeichnet, dass** die Markierungsmittel mindestens ein Markierungselement (16) aufweisen, wobei das mindestens eine Markierungselement den Auflageabschnitt (17) und den Markierungsabschnitt (18) aufweist.

3. Griff (1) nach Anspruch 2, **dadurch gekennzeichnet, dass** das mindestens eine Markierungselement (16) so gestaltet ist, dass es abnehmbar am Griff (1) angebracht werden kann.

4. Griff (1) nach einem der Ansprüche 2 oder 3, **dadurch gekennzeichnet, dass** der Griff (1) mindestens eine quer zur Längsrichtung des Griffs verlaufende durchgehende Aufnahme (14a-14b-14c) mit einem solchen Querschnitt aufweist, so dass sie geeignet ist, das mindestens eine Markierungselement (16) passgenau, aber lösbar aufzunehmen.

5. Griff (1) nach Anspruch 4, **dadurch gekennzeichnet, dass** der Griff (1) mindestens drei Durchgangsaufnahmen (14a-14b-14c) aufweist, die in einer Versatzrichtung (D) parallel zur Längsrichtung des Griffs versetzt sind.

6. Griff (1) nach Anspruch 4 oder Anspruch 5, **dadurch gekennzeichnet, dass** der Griff (1) einen Vorsprung (13) aufweist, der die mindestens eine durchgehende Aufnahme (14a-14b-14c) aufweist.

7. Griff (1) nach einem der Ansprüche 2 bis 6, **dadurch gekennzeichnet, dass** das mindestens eine Markierungselement (16) einstückig ist und so konfiguriert ist, dass, wenn das mindestens eine Markierungselement (16) abnehmbar an dem Griff (1) angebracht ist, der an einer Femurraspel (2) befestigt ist, die im Markkanal eines Oberschenkelknochens (30) positioniert ist, die Längsachse des mindestens einen Markierungselements (16) im Wesentlichen senkrecht zur Achse des Oberschenkelknochens (30) verläuft.

8. Griff (1) nach Anspruch 7, **dadurch gekennzeichnet, dass** das mindestens eine Markierungselement (16) einen kreisförmigen Querschnitt aufweist.

9. Greifstiel (1) nach Anspruch 6, **dadurch gekennzeichnet, dass** das mindestens eine Markierungselement (16) im Wesentlichen eine L-Form aufweist, die einen greifbaren Abschnitt (19) aufweist.

10. Instrument (10) zur Präparation eines Oberschenkelknochens (30), **dadurch gekennzeichnet, dass** es einen Griff (1) nach einem der Ansprüche 1 bis 9 und eine Oberschenkelraspel (2) aufweist.

## Claims

1. Gripping handle (1) intended to be assembled with a femoral rasp (2), the gripper handle (1) capable of being gripped by a physician to imprint a movement allowing the femoral rasp to rasp a medullary canal of a femur and comprising, arranged on it, means for materializing the position of the femoral rasp (2) relative to the femur, these materialization means comprising at least one support portion (17) capable of coming opposite the vertex (31) of the greater trochanter and at least one materialization portion (18) capable of being immobilized relative to the gripping handle in the longitudinal direction of this handle, so as to materialize at least one point belonging to the tangent plane (P) at the top of the greater trochanter when the support portion (17) rests on the summit (31) of the greater trochanter.

2. Gripping handle (1) according to claim 1, **characterized in that** the materialization means comprise at least one marker member (16), said at least one marker member comprising the support portion (17) and the portion materialization (18).

3. Gripping handle (1) according to claim 2, **characterized in that** said at least one marker member (16) is configured to be removably mounted on the gripping handle (1).

4. Gripping handle (1) according to either claim 2 or 3, **characterized in that** the gripping handle (1) comprises at least one through housing (14a-14b-14c) transverse to the longitudinal direction of the gripping handle (1), having a cross section such that it is capable of receiving said at least one marker member (16) in an adjusted but removable manner.

5. Gripping handle (1) according to claim 4, **characterized in that** the gripping handle (1) comprises at least three through housings (14a-14b-14c), offset in an offset direction (D) parallel to the longitudinal direction of the gripping handle (1).

6. Gripping handle (1) according to either claim 4 or claim 5, **characterized in that** the gripping handle (1) includes a boss (13) which comprises said at least one through housing (14a-14b-14c).

7. Gripping handle (1) according to any one of claims 2 to 6, **characterized in that** said at least one marker member (16) is in one piece and configured such that, when said at least one marker member (16) is removably mounted on the gripping handle (1) fixed to a femoral rasp (2) positioned in the medullary canal of a femur (30), the longitudinal axis of said at least one marker member (16) is substantially perpendicular to the axis of the femur (30).

8. Gripping handle (1) according to claim 7, **characterized in that** said at least one marker member (16) has a circular cross section.

9. Gripping handle (1) according to claim 6, **characterized in that** said at least one marker member (16) has substantially an L shape which comprises a grippable portion (19).

10. Instrument for preparing (10) a femur (30) **characterized in that** it comprises a gripping handle (1) according to any one of claims 1 to 9 and a femoral rasp (2).
